# FASCICULE DE BREVET EUROPEEN

(11) **EP 0 701 128 B1**
(45) Date de publication et mention de la délivrance du brevet: **05.12.2001**
(21) Numéro de dépôt: 95402003.8
(22) Date de dépôt: 04.09.1995
(51) Int. Cl.: G01N 33/24, G01N 15/08, G01N 27/04

(54) **Dispositif de mesure pétrophysique et méthode de mise en oeuvre**
Petrochemische Messvorrichtung und Verfahren zur Durchführung diesen Messungen
Apparatus for petrophysical measurements and method for carrying out the same

(30) Priorité: 09.09.1994 FR 9410783
(43) Date de publication de la demande: 13.03.1996
(73) Titulaire: INSTITUT FRANCAIS DU PETROLE, 92500 Rueil Malmaison (FR)
(72) Inventeur: Longeron, Daniel, F-78500 Sartrouville (FR); Fleury, Marc, F-78170 La Celle-Saint-Cloud (FR)

(56) Documents cités:
- FR-A- 2 568 373
- US-A- 2 534 718
- US-A- 4 734 649
- US-A- 4 907 448
- US-A- 5 297 420

## Description

La présente invention concerne un dispositif pour faire des mesures pétrophysiques permettant de faire tout à la fois des mesures de la pression capillaire régnant dans un échantillon géologique et des mesures de sa conductivité électrique. Un tel outil convient pour tester des échantillons géologiques et déterminer différents paramètres tels que la pression capillaire des roches dans des phases de drainage ou d'imbibition, leurs indices de mouillabilité, leurs perméabilités relatives, leurs indices de résistivité etc.

Le dispositif trouve des applications notamment dans le domaine pétrolier pour tester des roches qui ont été prélevés dans des formations recélant ou susceptibles de recéler des effluents pétroliers.

Il est important de déterminer la mouillabilité des roches vis à vis de l'eau et de l'huile qui peuvent y être contenues. A cet effet, il faut procéder à un draînage de la roche c'est-à-dire à un déplacement des fluides visant à diminuer la saturation en eau, suivi d'une imbibition, en désignant par ce terme un déplacement des fluides permettant d'augmenter la saturation en eau (Sw) de la roche. La pression capillaire en un point se définit comme la différence Pc à l'équilibre entre la pression Po de l'huile et celle Pw de l'eau. Ce paramètre n'a de sens que si les deux fluides sont en phase continue dans le milieu poreux. Pour un milieu mouillable à l'eau, seules les valeurs positives ont un sens. Par contre, lorsque le milieu a une mouillabilité mixte, les fluides peuvent rester en phase continue aussi bien pour les pressions capillaires (Pc) positives que négatives.

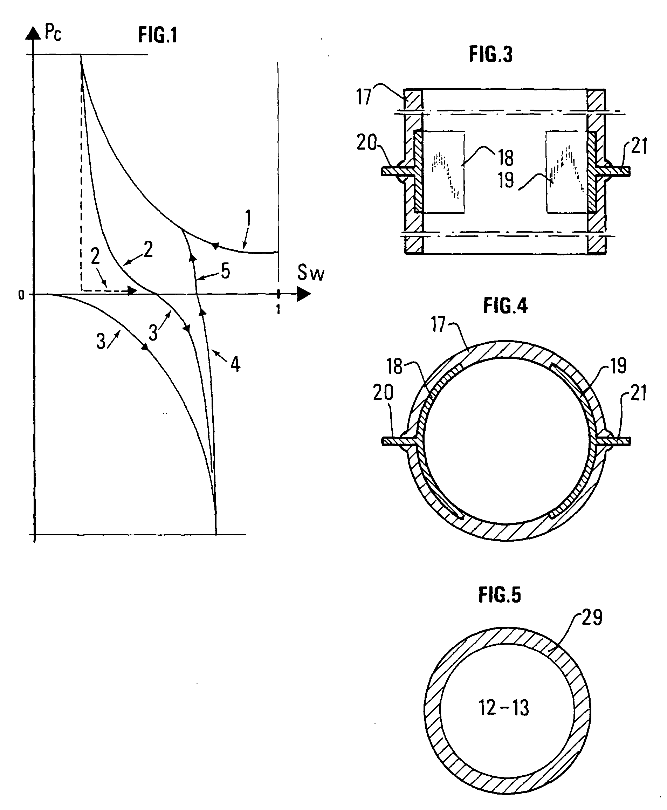

Pour une application de ce type, un cycle complet de mesure de la pression capillaire doit donc comporter (Fig. 1):
a) un draînage primaire positif d'un échantillon saturé initialement en eau à 100% (courbe 1);
b) une imbibition positive (courbe 2);
c) une imbibition négative (courbe 3);
d) un draînage négatif (courbe 4); et
e) un draînage secondaire positif (courbe 5).

La connaissance de différents paramètres et notamment de la mouillabilité des roches, est utile notamment quand on doit procéder à une récupération assistée d'une formation, en draînant les effluents qu'elle contient par une injection d'un fluide sous pression, et déterminer par des tests préalables, le fluide (eau ou gaz) qui convient le mieux pour déplacer les effluents.

L'invention trouve aussi des applications en génie civil pour faire de l'hydrologie de terrains pour évaluer leur degré de pollution par exemple, ou encore dans le bâtiment pour tester des matériaux de construction afin notamment de décider de traitements hydrofuges par exemple.

Il est connu de mesurer la pression capillaire de roches saturées de fluides en les soumettant à une centrifugation à vitesse progressive, et en mesurant la quantité de fluide produite en fonction de la vitesse. L'échantillon saturé de liquide est placé dans une enceinte dont l'axe est orienté suivant la direction de la force centrifuge et on injecte un autre fluide qui prend la place du fluide expulsé au fur et à mesure de son expulsion. Dans la phase de ré-imbibition, on diminue la vitesse de façon à étudier la réintégration du fluide initial dans l'échantillon. Le champ de pression créé par la centrifugation s'exprime en fonction de la densité r, du rayon R et de la vitesse angulaire w, par la relation : 1/2 w².r(Rmax²- R²), pour chaque fluide. On impose que la pression des deux fluides à la sortie de l'échantillon soit la même et qu'elle s'annule à la sortie. Les saturations locales, avec ce type de méthode, sont calculées par un programme d'inversion à partir de la quantité totale d'eau expulsée hors de l'échantillon. Cette méthode est mise en oeuvre par exemple dans les demandes de brevet FR-A-2 666 147 et EN. 92/15215 du demandeur.

Une autre méthode dite "dynamique", consiste à placer un échantillon dans une enceinte allongée terminée à ses deux extrémités par des membranes perméables à l'eau. A une première extrémité, on injecte directement de l'huile sous pression dans l'enceinte. De l'eau y est également injectée mais cette injection est effectuée au travers de la membrane et à une pression inférieure. A l'extrémité opposée, l'huile est évacuée directement alors que l'eau sort au travers de la membrane terminale. En réglant les débits d'injection d'huile et d'eau, on s'arrange pour que la pression capillaire soit la même à l'entrée de l'enceinte qu'à sa sortie, ce qui entraîne une saturation uniforme pouvant être déduite du bilan des fluides. La pression capillaire est obtenue par exemple en mesurant la différence entre les pressions de l'huile et de l'eau à la sortie de l'enceinte. Une telle méthode est décrite notamment par Brown H.W. in "Capillary Pressure Investigations" Petroleum Transaction AIME, vol. 192, 1951.

Par le brevet US 4 924 187, on connait une méthode pour réaliser des mesures de mouillabilité sur des échantillons de roche poreuse dans une cellule de confinement. Le barreau constituant l'échantillon à étudier, est placé à l'intérieur d'une gaîne déformable allongée associée à des moyens de pression pour exercer latéralement sur lui une pression de confinement. A une première extrémité, la cellule est fermée par une membrane poreuse perméable à un premier fluide telle qu'une saumure saturant le barreau mais non à un second fluide tel que de l'huile ou un gaz. La membrane poreuse est constituée d'une plaque de céramique. Ce fluide de déplacement est injecté sous pression à l'extrémité de la cellule opposée et le premier fluide, chassé hors de l'échantillon, est recueilli à la première extrémité. Entre les embouts conducteurs fermant la cellule à ses deux extrémités, est appliqué une tension électrique. Au moyen d'électrodes traversant radialement la gaîne et au contact de l'échantillon à des emplacements différents de sa longueur, on mesure la résistance électrique inter-électrodes et ses variations au fur et à mesure que le fluide mouillant est draîné hors du barreau.

Le dispositif selon l'invention permet de réaliser sur un échantillon solide poreux mouillable par au moins un premier fluide, des phases de draînage et d'imbibition successives, de façon à déterminer des paramètres physiques, (notamment pétrophysiques): pressions capillaires, saturation en eau (Sw), etc. de cet échantillon. Le dispositif comporte une cellule de confinement comprenant au moins une paroi déformable et des moyens pour appliquer un fluide sous pression pour exercer sur l'échantillon une pression latérale déterminée, deux membranes semi-perméables délimitant longitudinalement la cellule de confinement, la première étant mouillable par un premier fluide conducteur, la deuxième étant mouillable par un deuxième fluide et non-mouillable par le premier fluide, et des moyens pour déplacer le premier et le deuxième fluide au travers de l'échantillon, suivant une première direction et suivant la direction opposée à cette première direction, ainsi que des moyens pour mesurer les variations de la conductivité inter-électrodes.

Il est caractérisé en ce qu'il comporte au moins une paire d'électrodes disposées en regard les unes des autres suivant au moins une section transversale de la cellule, ces électrodes étant associées à la paroi déformable et pressées fortement contre l'échantillon par application du fluide sous pression. .

Le dispositif comporte par exemple deux embouts coaxiaux entre lesquels est disposé l'échantillon, les membranes semi-perméables étant disposées contre des faces intérieures en regard l'une de l'autre de ces deux embouts. La gaîne déformable est par exemple associée extérieurement à ces deux embouts.

Les moyens pour appliquer un fluide sous pression comportent un corps extérieur rigide dans laquel est disposée la cellule de confinement, et des moyens de connexion de cette chambre à un ensemble délivrant un fluide sous pression.

Suivant un mode de réalisation, ce corps est délimité par exemplepar un élément tubulaire associé à ses deux extrémités opposées à deux flasques latéraux, coopérant chacun avec un des deux embouts.

Suivant un autre mode de réalisation, le corps est délimité par deux manchons appliqués l'un contre l'autre par l'intermédiaire de joints d'étanchéité, ces deux manchons coopérant chacun avec un des deux embouts, l'échantillon est placé à l'intérieur d'une pièce annulaire en élastomère formant la gaîne.

Les électrode sont connectées par exemple à un appareil de mesure de conductivité extérieur au corps par l'intermédiaire de conducteurs sortant du corps par des traversées étanches.

Les moyens pour obtenir les déplacements de fluides comportent par exemple une première source délivrant le premier fluide et une deuxième source délivrant le deuxième fluide, et des canaux dans les deux embouts pour faire communiquer leurs faces intérieures en regard respectivement avec la première source et la deuxième source

De préférence, les faces intérieures en regard des deux embouts sont pourvues chacune d'un réseau de rainures.

Chaque membrane semi-perméable comporte par exemple une partie périphérique adaptée à empêcher toute fuite périphérique de fluide.

Suivant un mode de réalisation, le dispositif comporte une plaque pourvue d'une partie centrale perforée et d'une partie extérieure pleine, disposée entre chaque membrane semi-perméable et l'embout correspondant, et des joints d'étanchéité pour empêcher les fuites de fluide par contournement des membranes.

Suivant un mode de réalisation particulièrement avantageux, les embout sont dimensionnés de façon à confiner un échantillon dont l'épaisseur est inférieure à la section transversale.

Le dispositif peut aussi comporter une enceinte thermo-régulée adaptée à contenir la cellule de confinement.

Suivant un autre mode de réalisation, le dispositif comporte au moins une première paire d'électrodes pour l'application d'une différence de potentiel et au moins une deuxième paire d'électrodes pour la mesure du courant électrique traversant l'échantillon.

La méthode selon l'invention est caractérisée en ce qu'elle comporte le confinement de l'échantillon dans une cellule de confinement délimitée par une gaîne déformable associée à des moyens d'application de fluide sous pression pour exercer sur l'échantillon une pression de confinement déterminée, et deux membranes semi-perméables perméables respectivement à un premier fluide et à une deuxième fluide, et à déplacer les fluides dans l'échantillon au travers des deux membranes, tout en mesurant la variation de la conductivité de l'échantillon au moyen d'électrodes disposées au contact de l'échantillon, suivant au moins une section transversale de la cellule, et des moyens pour établir un champ électrique entre les électrodes et pour mesurer les variations de la conductivité inter-électrodes.

Le dispositif selon l'invention permet d'accélérer de façon considérable la vitesse des analyses d'échantillons, sans altérer pour autant la représentativité des mesures effectuées. Cela est dû à l'utilisation d'une cellule adaptée à des échantillons d'épaisseurs relativement faibles, de membranes de préférence micro-poreuses, qui permettent d'atteindre plus rapidement un état d'équilibre à chaque palier de pression.

C'est dû également à l'utilisation d'électrodes plus enveloppantes facilitant un meilleur suivi des variations de la conductivité de l'échantillon. Les mesures de conductivité électrique de l'échantillon en fonction de la saturation, sont particulièrement représentatives du fait que les électrodes sont fortement pressées contre l'échantillon. La forme enveloppante des électrodes permet en outre une mesure bien représentative également du facteur de formation.

Des exemples non limitatifs de réalisation du dispositif selon l'invention, va être décrit ci-après en se référant aux dessins annexés où :
- la Fig. 1 montre à titre indicatif les variations que subit la pression capillaire dans un échantillon au cours d'un cycle complet de draînage-imbibition;
- la Fig.2 montre schématiquement en coupe longitudinale un premier mode de réalisation du dispositif;
- la Fig.3 montre schématiquement en coupe transversale, la gaîne de confinement de l'échantillon avec les électrodes;
- la Fig.4 montre schématiquement en coupe transversale, la même gaîne de confinement;

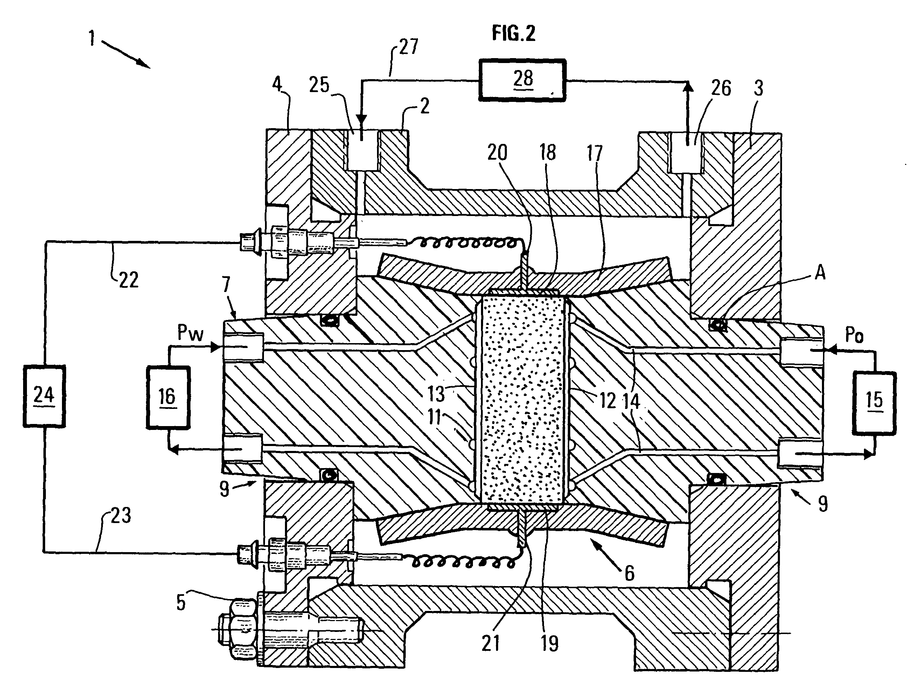

- la Fig.5 montre schématiquement une membrane micro-poreuse avec des moyens d'étanchéité périphériques;
- les Fig.6 et 7 montrent schématiquement en coupe longitudinale un deuxième mode de réalisation du dispositif et un détail de celui-ci; et
- la Fig.8 montre une coupe transversale de ce deuxième mode.

Le dispositif selon l'invention comporte un corps creux 1 délimité constitué d'une partie cylindrique 2 fermée à ses extrémités, par deux flasques 3, 4. Des moyens de fixation 5 et des joints (non représentés) permettent de solidariser les deux flasques 3, 4 à la partie cylindrique 2.

A l'intérieur du corps, est placée une cellule 6 de confinement comportant deux embouts cylindriques 7, 8. pourvus extérieurement de joints d'étanchéité A qui sont engagés respectivement dans des ouvertures 9 ménagées dans les deux flasques 3, 4, suivant leur axe central. L'échantillon à étudier est placé entre eux. Les faces terminales en regard l'une de l'autre des deux embouts, comportent chacune un réseau de rainures 11. Contre la face de l'un des embouts, est appliquée une première membrane semi-perméable 12 perméable à un premier fluide (une saumure telle qu'on en trouve dans les échantillons géologiques par exemple). Contre la face de l'autre est appliquée une autre membrane semi-perméable 13, perméable à un deuxième fluide tel que de l'huile. On utilise de préférence des cloisons micro-poreuses fabriquées par exemple par les firmes Gore Tex, Sartorius, Poretics, Millipore etc. Des canaux 14 traversent l'embout 7 et font communiquer le réseau de rainures 11 sur sa face terminale, avec une première source 15 délivrant le premier fluide sous pression. De même, des canaux 14 traversent l'embout 8 et font communiquer le réseau de rainures 11 correspondant avec une deuxième source 16 délivrant le deuxième fluide sous pression.

La partie de chaque embout 7, 8 à l'intérieur du corps est par exemple de forme tronconique. Une gaîne souple 17 en élastomère par exemple est enfilée sur les parties tronconiques des deux embouts pour confiner l'espace où se trouve l'échantillon. Dans sa partie centrale (Fig.2, 3), la gaîne souple moule deux électrodes 18, 19 chacune en forme de portion de cercle, disposées de préférence symétriquement par rapport à l'échantillon et en contact électrique avec lui. Chacune couvre par exemple un angle de 120°. Ces deux électrodes sont reliées à des conducteurs électriques 20, 21 traversant la gaîne souple 17. Par des traversées étanches 22, 23, ils sortent du corps 1 et sont reliés à un système de mesure de conductivité électrique 24 d'un type connu.

La partie cylindrique 2 du corps communique par des ouvertures 25, 26 et par l'intermédiaire d'un circuit 27, avec une source 28 délivrant un fluide de confinement sous pression. L'injection de ce fluide a pour effet de plaquer la gaîne 17 et les électrodes 18, 19 contre la périphérie de l'échantillon et permet de reproduire la pression auquel il était soumis là où il a été prélevé, dans le sous-sol par exemple.

On utilise par exemple des membranes semi-perméables 12, 13 pourvues sur leur pourtour (Fig.4) d'une zone 29 renforcée et adhésive, de façon à pouvoir être collée à la périphérie de l'échantillon et éviter toute fuite périphérique de fluide.

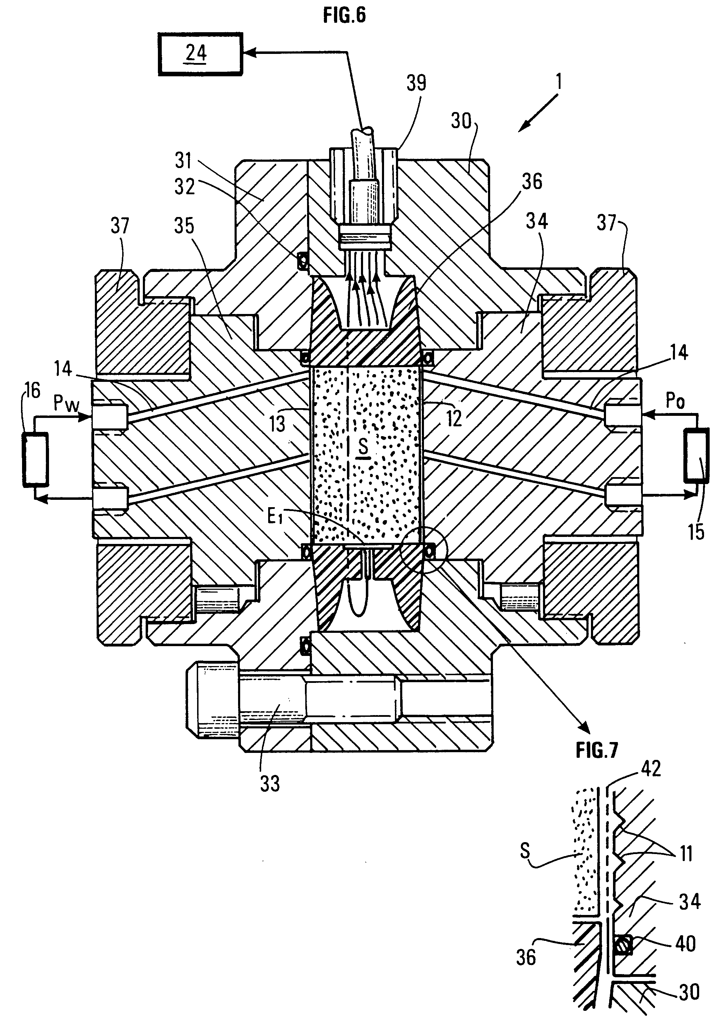

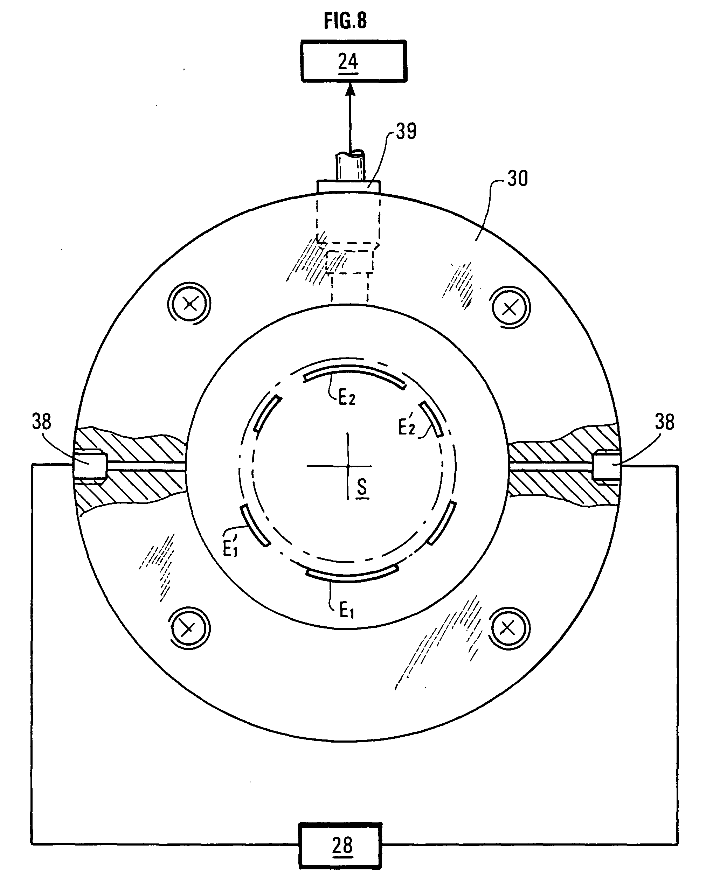

Suivant le mode de réalisation des Fig.6 à 8, le corps creux 1 est constitué de deux manchons 30, 31 à symétrie cylindrique. Ils sont appliqués l'un contre l'autre par l'intermédiaire de joints d'étanchéité 32 et réunis par des vis 33. Les deux manchons comportent chacun une cavité axiale pour un embout 34, 35. L'échantillon S est placé à l'intérieur d'une pièce annulaire en élastomère formant gaîne 36. L'ensemble de l'échantillon S et de la gaîne 36, est installé dans une cavité intérieure du manchon 31 et se trouve délimité axialement de part et d'autre par les deux embouts 34, 35, et au contact avec les membranes semi-perméables 12, 13. Les deux embouts 34, 35 dont les faces en regard l'une de l'autre sont pourvues de rainures 11, sont plaqués contre l'échantillon par vissage de deux écrous 37 dans les deux manchons 30, 31. Deux orifices radiaux 38 au travers de la paroi extérieure du manchon 30 (Fig.8) permettent l'application d'une pression de confinement axiale par la source de pression 28

Le dispositif comporte par exemple au moins un premier couple d'électrodes E1, E2 disposées à l'intérieur de la pièce annulaire 36, permettant l'application d'un courant électrique, et au moins un deuxième couple d'électrodes E'1, E'2 entre lesquelles on mesure la différence de potentiel créée en réponse à l'application du courant électrique. Cette affectation séparée des couples d'électrodes, l'un à l'application d'un courant, et l'autre, à la mesure de différences de potentiel, permet d'éviter les résistances dues aux contacts.

Au travers d'un bouchon 39 pourvu d'une traversée étanche, les fils connectés aux différentes électrodes, sont connectés au système de mesure de conductivité électrique 24.

Pour obtenir une parfaite étanchéité de l'enceinte de confinement de l'échantillon S, chaque embout 34, 35 comporte (Fig.7) une rainure dans sa paroi terminale pour un joint d'étanchéité 40, et on interpose entre chacune des membranes 12, 13 et l'embout correspondant 34, 35 une grille en métal recouverte d'un enduit plastique 41. Cette grille est pourvue à sa périphérie d'une couronne non perforée 42 qui empêche toute fuite de fluide par contournement des deux membranes 12, 13.

L'un ou l'autre des modes de réalisation qui viennent d'être décrits peut être placé dans une enceinte thermorégulée (non représentée) destiner à restituer la température de la formation géologique d'où l'échantillon a été extrait.

### FONCTIONNEMENT

Un échantillon poreux saturé avec un premier fluide tel qu'une saumure, est placé entre les manchons avec de part et d'autre les membranes semi-perméables 12, 13. On met en place la gaîne autour des deux manchons pour former la cellule de confinement. Le corps 1 est ensuite refermé autour de la cellule de façon étanche et l'on applique une pression de confinement à l'échantillon au moyen de la source de fluide sous pression 28.

Au moyen de la source de fluide 16, on établit tout d'abord une circulation d'un deuxième fluide, dans le circuit constitué des canaux 14 et du réseau de rainures 11 de l'embout 7 ou 35 de façon à bien le répartir sur toute la face antérieure de la membrane semi-perméable 13. On procède de la même manière avec la source 15 pour établir une continuité du premier fluide dans les canaux 14 de l'autre embout 8 ou 34 et au contact de l'autre membrane semi-perméable 12.

La pression de circulation Po du premier fluide imposée par la source 15 restant stable, on augmente la pression du deuxième fluide Pw jusqu'à un premier palier. Il pénètre dans l'échantillon au travers de la membrane 13 et l'on recueille dans le circuit opposé le premier fluide balayé hors de l'échantillon, ayant traversé l'autre membrane 12. On mesure la quantité d'eau ainsi expulsée et l'on enregistre également dans l'ensemble 24, la variation de la conductivité électrique produite par le déplacement des fluides à l'intérieur de l'échantillon.

Par paliers successifs, on augmente la pression de balayage Po, en enregistrant les quantités d'eau expulsées ainsi que les variations consécutives de la conductivité du barreau. Le draînage se poursuit jusqu'à atteindre une valeur limite de pression. On peut alors établir une courbe représentative des variations de la saturation Sw de l'échantillon en fonction de la pression capillaire imposée. (courbe 1 de la Fig.1 par exemple).

L'agencement symétrique du dispositif se prête pareillement à des opérations de réimbibition de l'échantillon. Une diminution de la pression Po de l'huile et une augmentation corrélative de la pression de l'eau Po, a pour effet un déplacement en sens inverse des deux fluides à l'intérieur de l'échantillon, l'eau réintégrant les pores de la roche au travers de la membrane mouillable 12, le fluide de balayage réintégrant le circuit dans l'embout 7 ou 35 au travers de la membrane micro-poreuse 13. On procède également en continu à des mesures des variations de la conductivité électrique consécutives à ces déplacements de fluides.

Par des cycles de draînages et réimbibitions successifs, on peut dresser ainsi toutes les courbes de variations de la Fig. 1.

## Revendications

1. Dispositif pour réaliser sur un échantillon solide poreux (S) mouillable par au moins un premier fluide, des phases de drainage et d'imbibition, de façon à déterminer des paramètres physiques de cet échantillon, tels que les perméabilités relatives ou la pression capillaire par exemple, comportant une cellule de confinement délimité par un corps et deux embouts opposés (7,8), une gaine déformable (17, 36) pour contenir l'échantillon, des moyens pour appliquer un fluide sous pression pour exercer sur l'échantillon dans sa gaine une pression latérale déterminée, deux membranes semi-perméables (12, 13) disposées respectivement entre des faces opposées de l'échantillon et les deux embouts, la première étant mouillable par un premier fluide conducteur, la deuxième étant mouillable par un deuxième fluide et non-mouillable par le premier fluide, des moyens pour déplacer le premier et le deuxième fluide au travers de l'échantillon, suivant une première direction et suivant la direction opposée à cette première direction, et des moyens de mesure des variations de la conductivité de l'échantillon comprenant une paire d'électrodes (E1, E2) disposées entre l'échantillon et la gaine, de façon à être plaquées contre l'échantillon par la pression latérale, **caractérisé en ce qu'**il comporte au moins une deuxième paires d'électrodes (E'1, E'2) disposées entre l'échantillon et la gaine de façon à être plaquées contre l'échantillon par la pression latérale, les électrodes de chaque paire étant disposées en regard les unes des autres, la première paire d'électrodes (E1, E2) étant connectées à une source de courant électrique, chaque deuxième paire d'électrodes (E'1, E'2) étant connectée aux dits moyens de mesure de la conductivité inter-électrodes.

2. Dispositif selon la revendication 1, **caractérisé en ce que** la gaine déformable (17) est associée extérieurement aux deux embouts (7, 8).

3. Dispositif selon la revendication 1 ou 2, **caractérisé en ce que** le corps rigide (1) est délimité par un élément tubulaire (2) associé à ses deux extrémités opposées à deux flasques latéraux (3, 4), coopérant chacun avec un des deux embouts (7,8).

4. Dispositif selon l'une des revendications précédentes, **caractérisé en ce que** les électrodes sont connectées à un appareil (24) de mesure de conductivité extérieur au corps par l'intermédiaire de conducteurs sortant du corps par des traversées étanches (22, 23, 39).

5. Dispositif selon l'une des revendications précédentes, **caractérisé en ce que** les moyens pour obtenir les déplacements de fluides comportent une première source (15) délivrant le premier fluide et une deuxième source (16) délivrant le deuxième fluide, et des canaux (14) dans les deux embouts (7, 8, 34, 35) pour faire, communiquer leurs faces intérieures en regard respectivement avec la première source (15) et la deuxième source (16).

6. Dispositif selon la revendication précédente, **caractérisé en ce que** les faces intérieures en regard des deux embouts (7, 8, 34, 35) sont pourvues chacune d'un réseau de rainures (11).

7. Dispositif selon l'une des revendications précédentes, **caractérisé en ce que** chaque membrane semi-perméable (12, 13) comporte une partie périphérique (29) adaptée à empêcher toute fuite périphérique de fluide.

8. Dispositif selon la revendication précédente, **caractérisé en ce que** chaque membrane semi-perméable (12, 13) comporte une partie périphérique renforcée et adhésive.

9. Dispositif selon l'une des revendications précédentes, **caractérisé en ce qu'**il comporte une grille pourvue à sa périphérie d'une couronne non perforée, interposée entre chacune des membranes (12, 13) et l'embout correspondant (34, 35), pour empêcher toute fuite de fluide par contournement des deux membranes (12, 13), les deux embouts comportent chacun une rainure dans sa paroi terminale pour un joint d'étanchéité (40).

10. Dispositif selon l'une des revendications précédentes, **caractérisé en ce que** les membranes semi-perméables (12, 13) sont des membranes micro-poreuses de préférence de faible épaisseur.

11. Dispositif selon l'une des revendications précédentes, **caractérisé en ce que** les embouts (7, 8, 34, 35) sont dimensionnés de façon à confiner un échantillon (S) dont l'épaisseur est inférieure à la section transversale.

12. Dispositif selon l'une des revendications précédentes, **caractérisé en ce qu'**il comporte en outre une enceinte thermo-régulée adaptée à contenir la cellule de confinement.

13. Méthode pour réaliser sur un échantillon solide poreux mouillable par au moins un premier fluide, des phases de drainage et d'imbibition, de façon à déterminer des paramètres physiques de cet échantillon tels que les perméabilités, relatives ou la pression capillaire par exemple, **caractérisée en ce qu'**elle comporte le confinement de l'échantillon dans une cellule de confinement délimitée par un corps et deux embouts opposés (7,8), une gaine déformable (17, 36) pour contenir l'échantillon, des moyens pour appliquer un fluide sous pression pour exercer sur l'échantillon dans sa gaine une pression latérale déterminée, deux membranes semi-perméables (12, 13) disposées respectivement entre des faces opposées de l'échantillon et les deux embouts, la première étant mouillable par un premier fluide conducteur, la deuxième étant mouillable par un deuxième fluide et non-mouillable par le premier fluide, des moyens pour déplacer le premier et le deuxième fluide au travers de l'échantillon, suivant une première direction et suivant la direction opposée à cette première direction, et des moyens de mesure des variations de la conductivité de l'échantillon comprenant une paire d'électrodes (E1, E2) disposées entre l'échantillon et la gaine, de façon à être plaquées contre l'échantillon par la pression latérale, tout en mesurant la conductivité de l'échantillon au moyen d'au moins deux paires d'électrodes toutes les deux disposées entre l'échantillon et la gaine de façon à être plaquées contre l'échantillon par la pression latérale, les électrodes de chaque paire étant disposées en regard les unes des autres, la première paire d'électrodes (E1, E2) étant connectées à une source de courant électrique, et chaque deuxième paire d'électrodes (E'1, E'2) étant connectée aux dits moyens de mesure de la conductivité inter-électrodes.

## Patentansprüche

1. Vorrichtung zur Vornahme von Phasen der Drainage und der Durchtränkung an einer porösen, festen Probe (S), die durch wenigstens ein erstes Fluid benetzbar ist, derart, dass die physikalischen Parameter dieser Probe bestimmt werden, so wie beispielsweise die relative Permeabilität oder der Kapillardruck, wobei die Vorrichtung eine abgeschlossene Zelle, begrenzt durch einen Körper und zwei gegenüberliegende Ansätze (7,8), eine verformbare Hülle (17,36) zur Aufnahme der Probe, Mittel zum Aufbringen eines Fluids unter Druck, um auf die Probe in der Hülle einen bestimmten lateralen Druck auszuüben, zwei semipermeable Membranen (12,13), jeweils angeordnet zwischen den gegenüberliegenden Flächen der Probe und den zwei Ansätzen, wobei der erste mit einem ersten leitenden Fluid benetzbar ist und der zweite mit einem zweiten Fluid benetzbar ist und nicht mit dem ersten Fluid benetzbar ist, ein Mittel zum Verdrängen des ersten und zweiten Fluids gemäß einer ersten Richtung und gemäß der der ersten Richtung entgegengesetzten Richtung durch die Probe hindurch, und Mittel zum Messen der Leitfähigkeitsänderung der Probe mit einem Elektrodenpaar (E1,E2) zwischen der Probe und der Hülle derart umfasst, dass sie durch lateralen Druck gegen die Probe gedrückt sind, **dadurch gekennzeichnet, dass** sie wenigstens ein zweites Elektrodenpaar (E'1,E'2) umfasst, das zwischen der Probe und der Hülle derart angeordnet ist, dass es durch lateralen Druck gegen die Probe gedrückt wird, wobei die Elektrodenpaare einander gegenüber angeordnet sind, und wobei das erste Elektrodenpaar (E1,E2) mit einer elektrischen Stromquelle verbunden ist, und jede des zweiten Elektrodenpaares (E'1,E'2) mit den Mitteln zur Messung der Leitfähigkeit zwischen den Elektroden verbunden ist.

2. Vorrichtung nach Anspruch 1, **dadurch gekennzeichnet, dass** die verformbare Hülle (17) äußerlich mit den beiden Ansätzen (7,8) verbunden ist.

3. Vorrichtung gemäß Anspruch 1 oder 2, **dadurch gekennzeichnet, dass** der steife Körper (1) begrenzt ist durch ein an seinen beiden gegenüberliegenden äußeren Enden zwei lateralen Flanschen (3,4) zugeordnetes röhrenförmiges Element (2), wobei jeder der Flansche mit einem der zwei Ansätze (7,8) zusammenwirkt.

4. Vorrichtung nach einem der vorhergehenden Ansprüche, **dadurch gekennzeichnet, dass** die Elektroden mit einem Gerät (24) zur Messung der Leitfähigkeit außerhalb des Körpers durch Leitungen, die den Körper über dichte Durchführungen verlassen (22,23,39), verbunden sind.

5. Vorrichtung gemäß einem der vorhergehenden Ansprüche, **dadurch gekennzeichnet, dass** die Mittel zum Erhalt der Verdrängung der Fluide eine erste Quelle (15) zur Lieferung des ersten Fluids und eine zweite Quelle (16) zur Lieferung des zweiten Fluids, und Kanäle (14) in den zwei Ansätzen (7,8,34,35) umfassen, um ihre sich gegenüberstehenden Innenflächen mit jeweils der ersten Quelle (15) und der zweiten Quelle (16) zu verbinden.

6. Vorrichtung gemäß einem der vorhergehenden Ansprüche, **dadurch gekennzeichnet, dass** die sich gegenüberliegenden Innenflächen der beiden Ansätze (7,8,34,35) jeweils mit Rillennetzen (11) versehen sind.

7. Vorrichtung gemäß einem der vorhergehenden Ansprüche, **dadurch gekennzeichnet, dass** jede semipermeable Membran (12,13) ein Umfangsteil (29) umfasst, angepasst, um das periphere Auslaufen des Fluids komplett zu verhindern.

8. Vorrichtung gemäß dem vorhergehenden Anspruch, **dadurch gekennzeichnet, dass** jede semipermeable Membran (12,13) einen peripher verstärkten und adhäsiven Teil umfasst.

9. Vorrichtung gemäß einem der vorhergehenden Ansprüche, **dadurch gekennzeichnet, dass** sie ein an seiner Peripherie mit einem nicht perforierten Aufsatzteil versehenes Gitter umfasst, angeordnet zwischen jeder der Membranen (12,13) und dem entsprechenden Ansatz (34,35), um den gesamten Abfluss von Fluid durch Umgehung der zwei Membranen (12,13) zu verhindern, wobei jeder der zwei Ansätze eine Nut in seiner Abschlusswand für eine Dichtung (40) umfasst.

10. Vorrichtung gemäß einem der vorhergehenden Ansprüche, **dadurch gekennzeichnet, dass** die semipermeablen Membranen (12,13) mikroporöse Membranen sind, vorzugsweise von geringer Dicke.

11. Vorrichtung gemäß einem der vorhergehenden Ansprüche, **dadurch gekennzeichnet, dass** die Ansätze (7,8,34,35) derart dimensioniert sind, dass sie eine Probe (S) einschließen, die eine Dicke kleiner als der Querschnitt aufweist.

12. Vorrichtung gemäß einem der vorhergehenden Ansprüche, **dadurch gekennzeichnet, dass** sie im Übrigen ein thermoreguliertes Gehäuse umfasst, das zur Aufnahme der abgeschlossenen Zelle angepasst ist.

13. Verfahren zur Vornahme von Phasen der Drainage und der Durchtränkung an einer porösen festen Probe, die durch wenigstens ein erstes Fluid benetzbar ist, derart, dass die physikalischen Parameter dieser Probe bestimmt werden, so wie beispielsweise die relative Permeabilität oder der Kapillardruck, **dadurch gekennzeichnet, dass** es das Einschließen der Probe in einer abgeschlossenen Zelle umfasst, begrenzt durch eine Körper und zwei gegenüberliegende Ansätze (7,8), eine verformbare Hülle (17,36) zur Aufnahme der Probe, Mittel zum Aufbringen eines Fluids unter Druck, um auf die Probe in der Hülle einen bestimmten lateralen Druck auszuüben, zwei semipermeable Membranen (12,13), jeweils angeordnet zwischen den gegenüberliegenden Flächen der Probe und den zwei Ansätzen, wobei der erste mit einem ersten leitenden Fluid benetzbar ist und der zweite mit einem zweiten Fluid benetzbar ist und nicht mit dem ersten Fluid benetzbar ist, ein Mittel zum Verdrängen des ersten und zweiten Fluids gemäß der ersten Richtung und gemäß der der ersten Richtung entgegengesetzten Richtung durch die Probe hindurch, und Mittel zum Messen der Leitfähigkeitsänderung der Probe mit einem Elektrodenpaar (E1,E2) zwischen der Probe und der Hülle derart umfasst, dass sie durch lateralen Druck gegen die Probe gedrückt werden, während die Leitfähigkeit der Probe mittels zweier Elektrodenpaare gemessen wird, wobei alle beiden zwischen der Probe und der Hülle derart angeordnet sind, dass sie durch lateralen Druck gegen die Probe gedrückt werden, wobei die Elektrodenpaare einander gegenüber angeordnet werden, und wobei das erste Elektrodenpaar (E1,E2) mit einer elektrischen Stromquelle verbunden wird, und jede des zweiten Elektrodenpaares (E'1,E'2) mit den Mitteln zur Messung der Leitfähigkeit zwischen den Elektroden verbunden wird.

## Claims

1. Device for subjecting a porous solid sample (S) wettable by at least a first fluid, to draining and soaking phases so as to determine physical parameters of that sample, such as relative permeabilities or capillary pressure for example, comprising a confinement cell delimited by a body and two opposing end pieces (7, 8), a deformable sheath (17, 36) to contain the sample, means for applying a fluid under pressure to exert a given lateral pressure on the sample in its sheath, two semi-permeable membranes (12, 13) disposed respectively between opposing faces of the sample and the two end pieces, the first being wettable by a first conducting fluid, the second being wettable by a second fluid and not wettable by the first fluid, means for displacing the first and the second fluid through the sample in a first direction and in the opposite direction to that first direction, and means for measuring the variations in the conductivity of the sample comprising a pair of electrodes (E1, E2) disposed between the sample and the sheath so as to be pressed against the sample by the lateral pressure, **characterised in that** it comprises at least a second pair of electrodes (E'1, E'2) disposed between the sample and the sheath so as to be pressed against the sample by the lateral pressure, the electrodes of each pair being disposed facing one another, the first pair of electrodes (E1, E2) being connected to a source of electrical current, each second pair of electrodes (E'1, E'2) being connected to said means for measuring the inter-electrode conductivity.

2. Device according to claim 1, **characterised in that** the deformable sheath (17) is associated externally with the two end pieces (7, 8).

3. Device according to claim 1 or 2, **characterised in that** the rigid body (1) is delimited by a tubular element (2) associated at its two opposing ends with two side flanges (3, 4), each co-operating with one of the two end pieces (7, 8).

4. Device according to one of the preceding claims, **characterised in that** the electrodes are connected to an apparatus (24) for measuring conductivity outside the body through the intermediary of conductors emerging from the body through sealed passages (22, 23, 39).

5. Device according to one of the preceding claims, **characterised in that** the means for obtaining the displacement of fluids comprise a first source (15) delivering the first fluid and a second source (16) delivering the second fluid, and channels (14) in the two end pieces (7, 8, 34, 35) to allow their facing internal faces to communicate respectively with the first source (15) and the second source (16).

6. Device according to the preceding claim, **characterised in that** the facing internal faces of the two end pieces (7, 8, 34, 35) are each provided with a system of grooves (11).

7. Device according to one of the preceding claims, **characterised in that** each semi-permeable membrane (12, 13) comprises a peripheral portion (29) adapted to prevent any peripheral leak of fluid.

8. Device according to the preceding claim, **characterised in that** each semi-permeable membrane (12, 13) comprises a reinforced and adhesive peripheral portion.

9. Device according to one of the preceding claims, **characterised in that** it comprises a grill provided on its periphery with a non-perforated ring, interposed between each of the membranes (12, 13) and the corresponding end piece (34, 35), to prevent any leak of fluid due to twisting of the two membranes (12, 13), the two end pieces each comprising a groove in its end wall for a seal (40).

10. Device according to one of the preceding claims, **characterised in that** the semi-permeable membranes (12, 13) are micro-porous membranes of preferably little thickness.

11. Device according to one of the preceding claims, **characterised in that** the end pieces (7, 8, 34, 35) are dimensioned so as to confine a sample (S) the thickness of which is less that the cross-section.

12. Device according to one of the preceding claims, **characterised in that** it additionally comprises a thermoregulated enclosure adapted to contain the confinement cell.

13. Method for subjecting a porous solid sample wettable by at least a first fluid, to draining and soaking phases so as to determine physical parameters of that sample such as relative permeabilities or capillary pressure for example, **characterised in that** it comprises confinement of the sample in a confinement cell delimited by a body and two opposing end pieces (7, 8), a deformable sheath (17, 36) to contain the sample, means for applying a fluid under pressure to exert a given lateral pressure on the sample in its sheath, two semi-permeable membranes (12, 13) disposed respectively between opposing faces of the sample and the two end pieces, the first being wettable by a first conducting fluid, the second being wettable by a second fluid and not wettable by the first fluid, means for displacing the first and the second fluid through the sample in a first direction and in the opposite direction to that first direction, and means for measuring the variations in the conductivity of the sample comprising a pair of electrodes (E1, E2) disposed between the sample and the sheath so as to be pressed against the sample by the lateral pressure, while measuring the conductivity of the sample by means of at least two pairs of electrodes, both disposed between the sample and the sheath so as to be pressed against the sample by the lateral pressure, the electrodes of each pair being disposed facing one another, the first pair of electrodes (E1, E2) being connected to a source of electrical current, and each second pair of electrodes (E'1, E'2) being connected to said means for measuring the inter-electrode conductivity.
